(19)

Europäisches
Patentamt
European
Patent Office
Office européen
des brevets

(11) **EP 4 485 354 A1**

(12) **EUROPEAN PATENT APPLICATION**

(43) Date of publication:
**01.01.2025 Bulletin 2025/01**

(21) Application number: **23194315.0**

(22) Date of filing: **30.08.2023**

(51) International Patent Classification (IPC):
**G06T 7/55** *(2017.01)*     **G06T 7/60** *(2017.01)*
*A45D 31/00* *(2006.01)*     *G01B 11/00* *(2006.01)*
*G06F 3/048* *(2013.01)*

(52) Cooperative Patent Classification (CPC):
**G06T 7/55; A45D 31/00; G06Q 30/0621;**
**G06T 7/60;** G01B 11/00; G06T 2207/20084;
G06T 2207/30196; G06V 40/11; G07F 17/04;
G16H 30/40; G16H 50/50

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **27.06.2023 US 202318215151**

(71) Applicant: **Standard Measure Technologies Corp.**
**Davie, FL 33314 (US)**

(72) Inventors:
- **Edwards, Aurelia**
  **Davie, FL 33314 (US)**
- **Lysyuk, Viktor**
  **Davie, FL 33314 (US)**
- **Rethage, Dario**
  **Davie, FL 33314 (US)**

(74) Representative: **Hahner, Ralph**
**Wallinger Ricker Schlotter Tostmann**
**Patent- und Rechtsanwälte Partnerschaft mbB**
**Zweibrückenstraße 5-7**
**80331 München (DE)**

(54) **METHOD, DEVICE, AND SYSTEM FOR PRECISION NAIL MEASUREMENT**

(57) Methods, systems, and computer program products to provide precision fingernail measurements. The method includes receiving first image data of a primary view of at least one fingernail and receiving second image data of a secondary view of at least one fingernail. The method also includes generating a three-dimensional (3D) digital nail model using the first and second image data. The method further includes determining a plurality of nail measurements based on the 3D digital nail model.

FIG. 9

**Description**

FIELD OF THE DISCLOSURE

The present disclosure relates generally to finger or toe nail (henceforth referred to as nail) measurement, and more specifically to a finger or toe nail measurement tool that uses machine vision and machine learning to detect and measure a user's fingernails.

BACKGROUND

Artificial nails, also known as press-on nail polish strips, nails, fake nails, false nails, fashion nails, acrylic nails, nail extensions nail enhancements, etc. are extensions placed over fingernails. Press-on nails may be made of various materials, and come in a variety of shapes, sizes, styles and colors. In order to accommodate various nail sizes, a set of press-on nails usually come with several sizes for each nail. For example, a set of artificial nails may include two nails of each size (e.g., size 0-9, some manufacturers are now offering 12-14 size options) with the hopes that ten nails out of the set of nails will fit the user. However, even by including the multiple different various sizes, the user may not achieve a good fit. Some users may have larger nails overall and only need sizes 5-10, similarly some users may have smaller nails and even the sizes 0-9 are not small enough to be a proper fit for all nails. Selling nail sets with extra nails also leads to unnecessary waste. In this regard, it is desirable to provide an improved solution which allows for precision nail measurement.

SUMMARY

Existing methods and systems do not provide sufficient precision of nail size measurement, which may lead to a suboptimal choice of artificial nails and likelihood that the artificial nails do not fit properly. The imprecision of existing nail measurement techniques may be a result of math errors, lack of certain necessary process steps (e.g., precise determination of the reference object edges), etc.
As shown in Table 1 - press-on nails come in standard sizes 0-9 that are correlated to a metric size in mm.

**Table 1. Standardized nail sizes**

| 0 | 1 | 2 | 3 | 4 | 5 | 6 | 7 | 8 | 9 |
|---|---|---|---|---|---|---|---|---|---|
| 17.5mm | 16mm | 15mm | 14mm | 13mm | 12mm | 11mm | 10mm | 9mm | 8mm |

Current sizing methods include:
Sizing kits - seller/designer sends the customer a sizing kit (e.g., all available sizes). The customer uses the sizing kit to measure their own nails and sends back the sizes. Example: Thumb-1, Pointer-5, Middle-4, Ring-5, Pinky-8 (assuming left/right hands same size). However, this method is prone to user error and also a lower user satisfaction due to the time required to send/receive the sizing kit before the user may order and receive their artificial nails.
Manual measurement - Customer uses clear tape to measure the widest part of their fingernail (cuticle sidewall to cuticle sidewall) and marks edges with a marker. The clear tape is then measured against measuring tape to get sizes in mm and then convert using the key above. This method is time consuming and is also prone to user error.
Embodiments of the present disclosure include a computer-implemented method for providing fingernail measurements, the computer-implemented method comprising: receiving first image data of a primary view of at least one fingernail; receiving second image data of a secondary view of the at least one fingernail; generating a three-dimensional (3D) digital nail model using the first and second image data; and determining a plurality of nail measurements based on the 3D digital nail model.
Embodiments of the present disclosure herein include, wherein the first and second image data is received using a camera in a mobile device, and wherein the first and second image data is received in the form of a video or a plurality of images at different angles and at different distances relative to the camera.
Embodiments of the present disclosure herein include, wherein the 3D digital nail model comprises a set of 3D points to represent the at least one fingernail, and wherein the plurality of nail measurements are determined based on the plurality of pixels.
Embodiments of the present disclosure herein include, wherein the 3D digital nail model is generated using point cloud modeling.
Embodiments of the present disclosure herein include, wherein the primary view comprises a top-down view of at least one fingernail.

Embodiments of the present disclosure herein include, wherein the secondary view comprises a view somewhere between, but neither a top-down or straight-on view of the nail plate of at least one fingernail.

Embodiments of the present disclosure herein include, wherein the first and second image data are taken with a reference object.

Embodiments of the present disclosure herein include, wherein the reference object comprises a standard-sized object such as a credit card, a coin, etc.

Embodiments of the present disclosure herein include, wherein the plurality of nail measurements comprises a curvature of a nail plate of at least one fingernail.

Embodiments of the present disclosure herein include, wherein the plurality of nail measurements comprises a shape of a nail plate of at least one fingernail.

Embodiments of the present disclosure herein include, displaying the plurality of nail measurements to a user interface of a mobile device running the native mobile application.

Embodiments of the present disclosure herein include, transmitting the plurality of nail measurements to another device.

Embodiments of the present disclosure herein include a computer-implemented method for providing fingernail measurements via a native mobile application, the computer-implemented method comprising: receiving first image data of a primary view of at least one fingernail; receiving second image data of a secondary view of the at least one fingernail; generating a three-dimensional (3D) digital nail model using the first and second image data; and determining a plurality of nail measurements based on the 3D digital nail model.

Embodiments of the present disclosure herein include a mobile device comprising: memory; a camera; and a processor, when executing a mobile application, the mobile device configured to: obtain first image data of a primary view of a user's fingernail nail plate; obtain second image data of a secondary view of the user's fingernail nail tip; generate a three-dimensional (3D) digital nail model using the first and second image data; and determine a plurality of nail measurements based on the 3D digital nail model.

Embodiments of the present disclosure herein include a user interface (UI) for nail measurement as described herein.

Embodiments of the present disclosure herein include a mobile application for nail measurement as described herein.

Embodiments of the present disclosure herein include a device for nail measurement as described herein.

Embodiments of the present disclosure herein include a nail measurement method as described herein.

Embodiments of the present disclosure herein include instructions provided to the user via the UI for taking multiple images (e.g., 4 fingers of left-hand, left-hand thumb, 4 fingers of right-hand, and right thumb).

Embodiments of the present disclosure herein include determining fingernail curvature and incorporating nail curvature into the measurement model to improve accuracy.

The phrases "at least one," "one or more," and "and/or," as used herein, are open-ended expressions that are both conjunctive and disjunctive in operation. For example, each of the expressions "at least one of A, B and C," "at least one of A, B, or C," "one or more of A, B, and C," "one or more of A, B, or C," and "A, B, and/or C" means A alone, B alone, C alone, A and B together, A and C together, B and C together, or A, B and C together.

The term "a" or "an" entity, as used herein, refers to one or more of that entity. As such, the terms "a" (or "an"), "one or more" and "at least one" can be used interchangeably herein.

The transitional term "comprising" is synonymous with "including," "containing," or "characterized by," is inclusive or open-ended and does not exclude additional, unrecited elements or method steps.

Unless otherwise indicated, all numbers expressing quantities, dimensions, conditions, ratios, ranges, and so forth used in the specification and claims are to be understood as being modified in all instances by the term "about" or "approximately". Accordingly, unless otherwise indicated, all numbers expressing quantities, dimensions, conditions, ratios, ranges, and so forth used in the specification and claims may be increased or decreased by approximately 5% to achieve satisfactory results. Additionally, where the meaning of the terms "about" or "approximately" as used herein would not otherwise be apparent to one of ordinary skill in the art, the terms "about" and "approximately" should be interpreted as meaning within plus or minus 5% of the stated value.

All ranges described herein may be reduced to any sub-range or portion of the range, or to any value within the range without deviating from the invention. For example, the range "5 to 55" includes, but is not limited to, the sub-ranges "5 to 20" as well as "17 to 54."

The transitional phrase "consisting of' excludes any element, step, or ingredient not specified in the claim, but does not exclude additional components or steps that are unrelated to the disclosure such as impurities ordinarily associated therewith.

The transitional phrase "consisting essentially of" limits the scope of a claim to the specified materials or steps and those that do not materially affect the basic and novel characteristic(s) of the claimed invention.

The use of "including," "comprising," or "having" and variations thereof herein is meant to encompass the items listed thereafter and equivalents thereof as well as additional items. Accordingly, the terms "including," "comprising," or "having" and variations thereof can be used interchangeably herein.

The preceding is a simplified summary of the disclosure intended to provide an understanding of some aspects of the settler devices of this disclosure. This Summary is neither an extensive nor exhaustive overview of the invention and its various aspects, embodiments, and configurations. It is intended neither to identify key or critical elements of the disclosure nor to delineate the scope of the disclosure but to present selected concepts of the disclosure in a simplified form as an introduction to the more detailed description presented below. As will be appreciated, other aspects, embodiments, and configurations of the disclosure are possible utilizing, alone or in combination, one or more of the features set forth above or described in detail below. As will be appreciated, other embodiments are possible using, alone or in combination, one or more of the features set forth above or described herein. For example, it is contemplated that various features and devices shown and/or described with respect to one embodiment may be combined with or substituted for features or devices of other embodiments regardless of whether or not such a combination or substitution is specifically shown or described herein. Additional aspects of the present invention will become more readily apparent from the Detailed Description, particularly when taken together with the drawings.

BRIEF DESCRIPTION OF THE DRAWINGS

Those of skill in the art will recognize that the following description is merely illustrative of the principles of the disclosure, which may be applied in various ways to provide many different alternative embodiments. This description is made for illustrating the general principles of the teachings of this disclosure and is not meant to limit the inventive concepts disclosed herein.

The accompanying drawings, which are incorporated in and constitute a part of the specification, illustrate embodiments of the disclosure and together with the general description of the disclosure given above and the detailed description of the drawings given below, serve to explain the principles of the disclosure.

It should be understood that the drawings are not necessarily to scale, and various dimensions may be altered. In certain instances, details that are not necessary for an understanding of the disclosure or that render other details difficult to perceive may have been omitted. It should be understood, of course, that the disclosure is not necessarily limited to the particular embodiments illustrated herein.

Figure 1 depicts various nail dimensions, in accordance with embodiments of the present disclosure;

Figure 2 depicts a landmark measurement method, in accordance with embodiments of the present disclosure;

Figure 3 depicts segmentation measurement method, in accordance with embodiments of the present disclosure;

Figure 4 depicts detection of a reference object, in accordance with embodiments of the present disclosure;

Figure 5 depicts various auxiliary views/nails curvature, in accordance with embodiments of the present disclosure;

Figure 6 depicts nail digital twin/3D model generation, in accordance with embodiments of the present disclosure;

Figure 7 depicts the two views that the method operates with, in accordance with embodiments of the present disclosure;

Figure 8 depicts correct and incorrect auxiliary view, in accordance with embodiments of the present disclosure;

Figure 9 depicts an example software architecture, in accordance with embodiments of the present disclosure;

Figure 10 illustrates an example of a system 100 for precision nail measurement in accordance with aspects of the present disclosure.

Figure 11 illustrates an example of a process flow 300 for performing precision nail measurement in accordance with aspects of the present disclosure.

Figure 12 illustrates an example configuration of network components that support the system

Figure 13 illustrates an example user interface instructing a user how to use the system.

DETAILED DESCRIPTION

Although the following text sets forth a detailed description of numerous different embodiments, it should be understood that the legal scope of the description is defined by the words of the claims set forth at the end of this disclosure. The detailed description is to be construed as exemplary only and does not describe every possible embodiment since describing every possible embodiment would be impractical, if not impossible. Numerous alternative embodiments could be implemented, using either current technology or technology developed after the filing date of this patent, which would still fall within the scope of the claims.

The ensuing description provides example aspects of the present disclosure, and is not intended to limit the scope, applicability, or configuration of the claims. Rather, the ensuing description will provide those skilled in the art with an enabling description for implementing the described examples. It being understood that various changes may be made in the function and arrangement of elements without departing from the spirit and scope of the appended claims. Various aspects of the present disclosure will be described herein with reference to drawings that are schematic illustrations of idealized configurations.

Aspects of the disclosure are further illustrated by and described with reference to apparatus diagrams, system diagrams, and flowcharts that relate to systems and methods for nail measurement.

As discussed above, in the manual/non-digital measurement method, the customer often uses clear tape to measure the widest part of their fingernail (cuticle sidewall to cuticle sidewall) and marks edges with a marker. The clear tape is then measured against measuring tape to get sizes in mm which are then converted to the standard sizes listed Table 1.

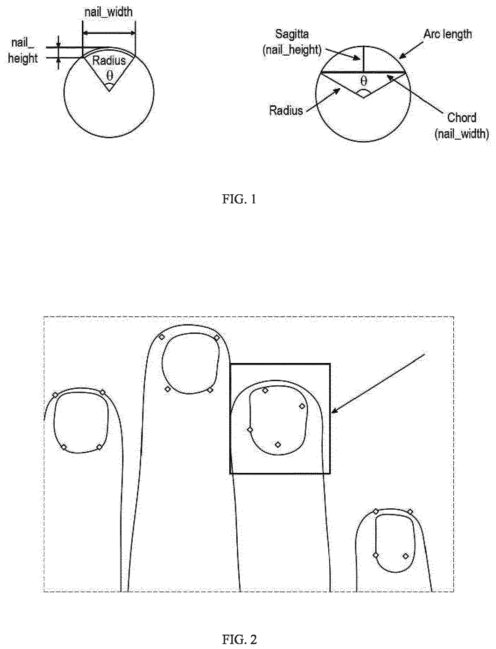

The present disclosure is directed to a digital measurement method to automatically determine the shape and absolute size of the nail to determine the nail measurements. In embodiments, the determined nail measurements may be correlated to the standard nail sizes in Table 1. In embodiments, a mobile (e.g., iOS, Android, etc.) app may be directed to providing more precise nail measurements, which can be used to determine the size of artificial nails. Figure 1 illustrates the various nail dimensions that may be determined using the present disclosure.

In embodiments machine vision is combined with AI to determine/measure nail width and nail height, which can then be used to determine the radius and nail size (using the formula below). The radius and nail size can then be mapped into the standardized sizes according to Table 1.

$$radius = \frac{sagitta}{2} + \frac{chord^2}{8*sagitta} = \frac{nail\_height}{2} + \frac{nail\_width^2}{8*nail\_height}$$

$$\theta = 2*arcsin\left(\frac{nail\_width}{2*radius}\right)$$

$$nail\_size = arc\_length = \theta*radius$$

Previous work employs a machine vision technique in which key points on the nail are found and then used to approximate the shape contour of the nail. Figure 2 illustrates a digital measurement method using landmarks. In Figure 2, the landmarks for one of the fingers has been recognized incorrectly, and therefore the corresponding nail has been also incorrectly oriented. This in turn would lead to a noticeable error in determining the size of the nail.

The landmark approach may also require the same hand/fingers to be measured several times, which may produce different results each time, which results in further inconsistencies. The issue of imprecise nail edge recognition is self-evident when the landmark detection results are plotted on the underlying image. The landmark approach uses only four landmarks for each nail; however, the four landmarks may not accurately represent the complex nail shape. This leads to a loss of a substantial amount of information which could be used to enhance the precision in determining the nail size.

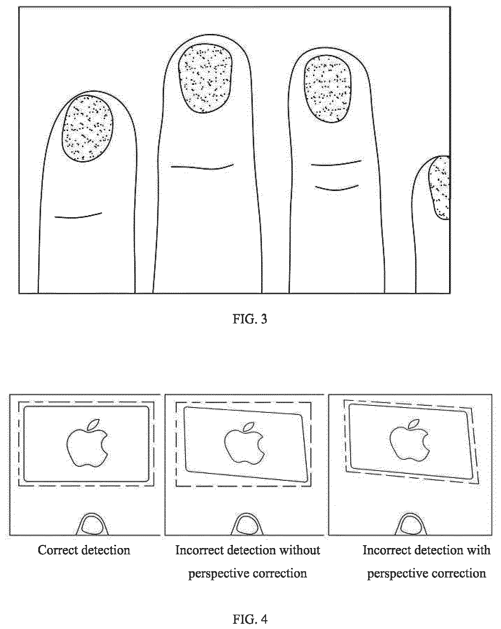

To address these shortcomings, we propose to abandon the landmark-based approach in favor of nail segmentation (illustrated in Figure 3), which, increases the precision of nail shape estimation. In segmentation, each pixel is classified on the basis of information about the neighboring pixels (context), which provides mutual error-correction, allowing for a higher degree of precision in the measurement process. Another benefit of segmentation is that, after the segmentation had been applied, the resulting output can be further refined with machine vision techniques. Test-time augmentation may be used to improve the precision of reference object detection (e.g., card edge detection). Test-time augmentation refers to the technique of running a detector on a series of augmented images created on the fly from the original input image, the series of augmented images is created by introducing small changes to the original input image. The series of augmented images may also be used to filter outliers.

Another way the present disclosure enhances measurement precision is by using a series of photographs taken from a varying distances and angles (e.g., multi-view processing) Figure 4 depicts the detection of a card as a reference object. The scaling of the nail shapes axis may not be accurate when the detection of the card boundaries is not accurate. If the card and/or camera is slightly turned, there will be errors in estimating the size of the objects in relation to the card shot in perspective projection, resulting a warped image.

To get a corrected image, the present disclosure uses edge detection techniques to correctly establish the coordinates of the card corners and then uses machine vision (e.g., Perspective Transform), to perform calculations on the basis of coordinates of the corners of the card.

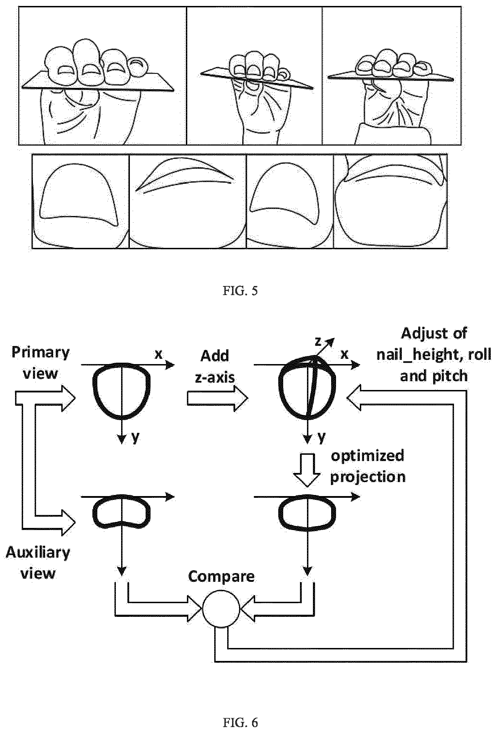

Considering the high degree of variance in people's nails (e.g., size, shape, curvature, length, etc.) and the fact that it would be practically impossible for different users to position their hands/fingers in the same position, or even the same user to position their hand/fingers in the same position in multiple pictures, as illustrated in Figure 5, the present disclosure uses a multistep iterative algorithm to provide precise nail size measurements.

The multi-step approach includes:

    1. Finger detector

    2. Nail segmentation model

    3. Nail digital twin (effectively, a 3D model of the nail)

In the process of optimization, the system varies a nail's digital twin's height (and respective curvature) and the nail's pose relative to the camera to ensure maximal similarity between the projection of the digital twin and the projection of the actual nail as depicted in the image. (See Figure 6). In simple terms, in the proposed process the digital 3D model of the nail is manipulated to obtain the image which resembles the actual nail photo as closely as possible. The nail's parameters obtained in this manner will then be used to determine the nail size.

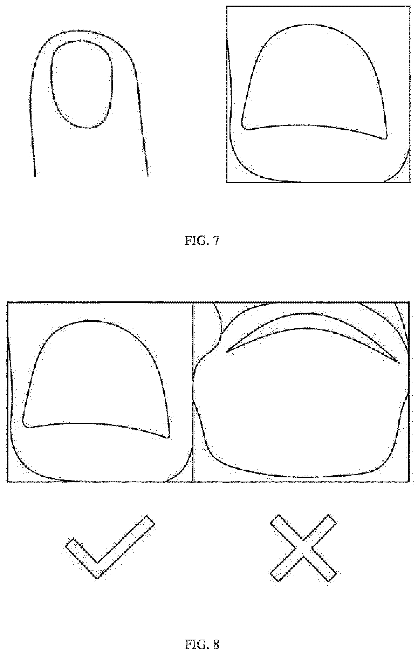

The present disclosure operates with two images (e.g., first and second image data), the main view and the auxiliary view as depicted in Figure 7.

Initially, the method segments the top-down view of the nail (e.g., main view). Segmented nail surface pixels will each have x and y coordinates, but the z coordinate will initially be equal zero (e.g., a flat nail surface).

A nail height (h) is determined for that view which forms the basis for the determination of z coordinate for each of the nail surface pixels.

$$z = h - r + sqrt(\, r*r\text{-}(x\text{-}w/2\text{-}0.5)**2),$$

where

$$r = (h/2) + ((w^{\wedge}2) / (8 * h)),$$

w - nail width
under the assumption that z - coordinate does not depend on y - coordinate.

The nail height (h) is determined, such that the subsequent selection of the pitch, roll and yaw of the obtained point cloud creates a projection which is the closest match with the segmented nail image in the auxiliary view. For ease of comparison, projection is scaled to have the same width with the segmented nail surface of the auxiliary view.

The present disclosure requires that the top of the nail is visible in both primary and auxiliary views. As a result, it is important to ask users not to rotate the camera fully to the point where the nail is completely perpendicular to the camera (i.e., right image in Figure 8).

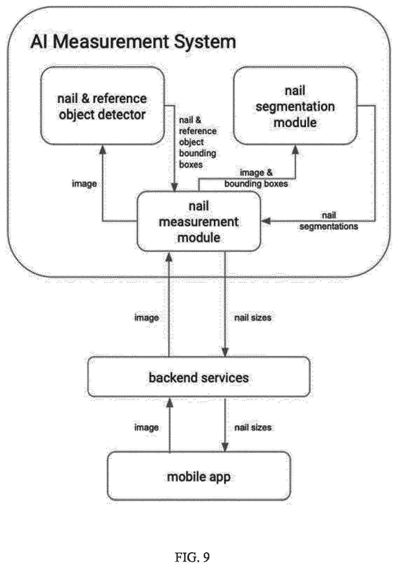

The method utilizes two AI models. This architecture requires data exchange between the two models via a local network (Figure 9).

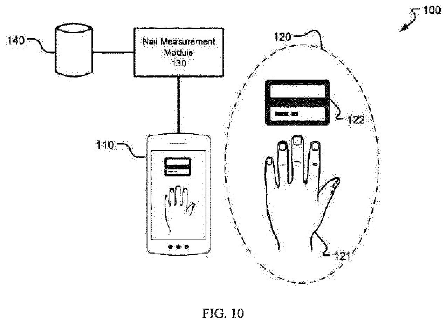

Figure 10 illustrates an example system 100 for providing precision nail measurements in accordance with aspects of the present disclosure. System 100 includes a mobile device 110, which captures an image of an area 120, the area 120 includes a user's hand 121 and a reference object 122. The image data is transmitted to the nail measurement module 130. In embodiments, the nail measurement module may comprise a mobile app on the mobile device 110. In embodiments, the mobile device 110 may include an Application Programming Interface (API) which communicates with the nail measurement module 130. The nail measurement module 130 may be stored in the cloud. The nail measurement module 130 may include and/or be connected to a database 140. The database 140 may store image data, measurement data, etc.

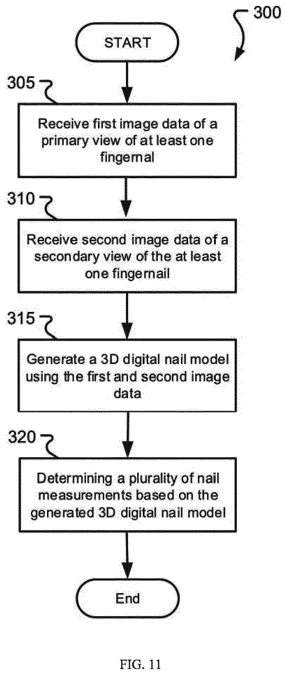

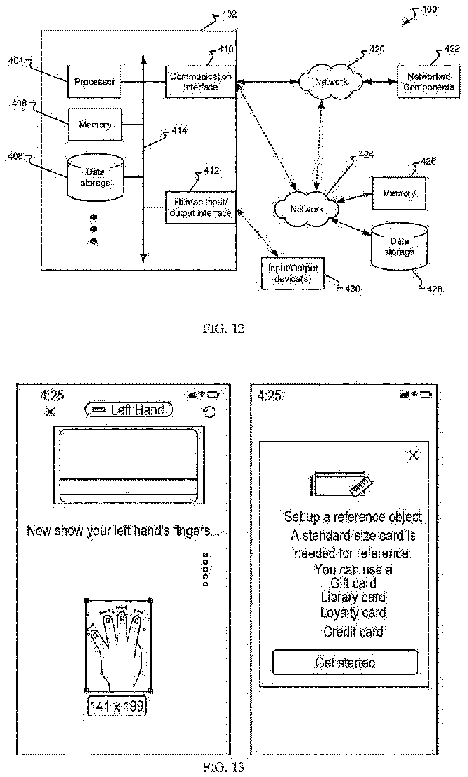

Figure 11 illustrates an example of a process flow 300 for precision nail measurement in accordance with aspects of the present disclosure. In some examples, process flow 300 may implement aspects of a nail measurement module 130 and/or a device 402 described with reference to Figures 10 and 12.

In the following description of the process flow 300, the operations may be performed in a different order than the order shown, or the operations may be performed in different orders or at different times. Certain operations may also be left out of the process flow 300, or other operations may be added to the process flow 300.

In step 305, first image data of a primary view of at least one fingernail is received. For example, using a camera on the mobile device 110 a main view (e.g., top down) of the 4 fingers of left-hand, the left-hand thumb, the 4 fingers of right-hand, and the right thumb may be recorded. More or fewer images may be captured. The first image data may comprise a still image or video data.

In step 310, second image data of a secondary view of at least one fingernail is received. For example, the auxiliary view (e.g., side view) of the 4 fingers of left-hand, the left-hand thumb, the 4 fingers of right-hand, and the right thumb). More or fewer images may be captured. The second image data may comprise a still image or video data.

In step 315, a three-dimensional (3D) digital nail model using the first and second image data is generated. For example, using point cloud a 3D nail model that is the closest match with the segmented nail image in the auxiliary view is generated.

In step 320, a plurality of nail measurements based on the 3D digital nail model are determined. The plurality of nail measurement may be correlated to the standardized nail sizes in Table 1.

Figure 12 depicts device 402 in system 400 in accordance with embodiments of the present disclosure. Device 402 may be an example of the mobile device 110 in Figure 10.

The components are variously embodied and may comprise processor 404. The term "processor," as used herein, refers exclusively to electronic hardware components comprising electrical circuitry with connections (e.g., pin-outs) to convey encoded electrical signals to and from the electrical circuitry. Processor 404 may be further embodied as a single electronic microprocessor or multiprocessor device (e.g., multicore) having electrical circuitry therein which may further comprise a control unit(s), input/output unit(s), arithmetic logic unit(s), register(s), primary memory, and/or other components that access information (e.g., data, instructions, etc.), such as received via bus 414, executes instructions, and outputs data, again such as via bus 414.

In other embodiments, processor 404 may comprise a shared processing device that may be utilized by other processes and/or process owners, such as in a processing array within a system (e.g., blade, multi-processor board, etc.) or distributed processing system (e.g., "cloud," farm, etc.). It should be appreciated that processor 404 is a non-transitory computing device (e.g., electronic machine comprising circuitry and connections to communicate with other components and devices). Processor 404 may operate a virtual processor, such as to process machine instructions not native to the processor (e.g., translate the VAX operating system and VAX machine instruction code set into Intel® 9xx chipset code to allow VAX-specific applications to execute on a virtual VAX processor), however, as those of ordinary skill understand, such virtual processors are applications executed by hardware, more specifically, the underlying electrical circuitry and other hardware of the processor (e.g., processor 404). Processor 404 may be executed by virtual processors, such as when applications (i.e., Pod) are orchestrated by Kubernetes. Virtual processors allow an application to be presented with what appears to be a static and/or dedicated processor executing the instructions of the application, while underlying non-virtual processor(s) are executing the instructions and may be dynamic and/or split among a number of processors.

In addition to the components of processor 404, device 402 may utilize memory 406 and/or data storage 408 for the storage of accessible data, such as instructions, values, etc. Communication interface 410 facilitates communication with components, such as processor 404 via bus 414 with components not accessible via bus 414. Communication interface 410 may be embodied as a network port, card, cable, or other configured hardware device. Additionally, or alternatively, human input/output interface 412 connects to one or more interface components to receive and/or present information (e.g., instructions, data, values, etc.) to and/or from a human and/or electronic device. Figure 13 depicts an example human interface, directing the user to capture an image of the hand.

Examples of input/output device(s) 430 that may be connected to an input/output interface include, but are not limited to, keyboard, mouse, trackball, printers, displays, sensor, switch, relay, speaker, microphone, still and/or video camera, etc. In another embodiment, communication interface 410 may comprise, or be comprised by, human input/output interface 412. Communication interface 410 may be configured to communicate directly with a networked component or utilize one or more networks, such as network 420 and/or network 424. Input/output device(s) 430 may be accessed by processor 404 via human input/output interface 412 and/or via communication interface 410 either directly, via network 424 (not shown), via network 420 alone (not shown), or via networks 424 and 420 (not shown).

Networks 420 and 424 may be a wired network (e.g., Ethernet), wireless (e.g., Wi-Fi, Bluetooth, cellular, etc.) network, or combination thereof and enable device 402 to communicate with networked component(s) 422 (e.g., automation system). In other embodiments, networks 420 and/or 424 may be embodied, in whole or in part, as a telephony network (e.g., public switched telephone network (PSTN), private branch exchange (PBX), cellular telephony network, etc.).

Components attached to network 424 may include memory 426 and data storage 428. For example, memory 426 and/or data storage 428 may supplement or supplant memory 406 and/or data storage 408 entirely or for a particular task or purpose. For example, memory 426 and/or data storage 428 may be an external data repository (e.g., server farm, array, "cloud," etc.) and allow device 402, and/or other devices, to access data thereon. Each of memory 406 and data storage 408, memory 426, data storage 428 comprise a non-transitory data storage comprising a data storage device.

Any of the steps, functions, and operations discussed herein can be performed continuously and automatically.

The exemplary apparatuses, systems, and methods of this disclosure have been described in relation to examples of a telemetry collection device 115. However, to avoid unnecessarily obscuring the present disclosure, the preceding description omits a number of known structures and devices. This omission is not to be construed as a limitation of the scope of the claimed disclosure. Specific details are set forth to provide an understanding of the present disclosure. It should, however, be appreciated that the present disclosure may be practiced in a variety of ways beyond the specific detail set forth herein.

It will be appreciated from the descriptions herein, and for reasons of computational efficiency, that the components of devices and systems described herein can be arranged at any appropriate location within a distributed network of components without impacting the operation of the device and/or system.

Unless otherwise defined, all terms (including technical and scientific terms) used herein have the same meaning as commonly understood by one of ordinary skill in the art to which this disclosure belongs. It will be further understood that terms, such as those defined in commonly used dictionaries, should be interpreted as having a meaning that is consistent with their meaning in the context of the relevant art and this disclosure.

While the flowcharts have been discussed and illustrated in relation to a particular sequence of events, it should be appreciated that changes, additions, and omissions to this sequence can occur without materially affecting the operation of the disclosed examples, configuration, and aspects.

The foregoing discussion of the disclosure has been presented for purposes of illustration and description. The foregoing is not intended to limit the disclosure to the form or forms disclosed herein. In the foregoing Detailed Description for example, various features of the disclosure are grouped together in one or more examples, configurations, or aspects for the purpose of streamlining the disclosure. The features of the examples, configurations, or aspects of the disclosure may be combined in alternate examples, configurations, or aspects other than those discussed above. This method of disclosure is not to be interpreted as reflecting an intention that the claimed disclosure requires more features than are expressly recited in each claim. Rather, as the following claims reflect, inventive aspects lie in less than all features of a single foregoing disclosed example, configuration, or aspect. Thus, the following claims are hereby incorporated into this Detailed Description, with each claim standing on its own as a separate preferred example of the disclosure.

Other variations are within the spirit of present disclosure. Thus, while disclosed techniques are susceptible to various modifications and alternative constructions, certain illustrated examples thereof are shown in drawings and have been described above in detail. It should be understood, however, that there is no intention to limit disclosure to specific form or forms disclosed, but on contrary, intention is to cover all modifications, alternative constructions, and equivalents falling within spirit and scope of disclosure, as defined in appended claims.

Use of terms "a" and "an" and "the" and similar referents in context of describing disclosed examples (especially in context of following claims) are to be construed to cover both singular and plural, unless otherwise indicated herein or clearly contradicted by context, and not as a definition of a term. Terms "comprising," "having," "including," and "containing" are to be construed as open-ended terms (meaning "including, but not limited to,") unless otherwise noted. "Connected," when unmodified and referring to physical connections, is to be construed as partly or wholly contained within, attached to, or joined together, even if there is something intervening. Recitation of ranges of values herein are merely intended to serve as a shorthand method of referring individually to each separate value falling within range, unless otherwise indicated herein and each separate value is incorporated into specification as if it were individually recited herein. In at least one example, use of the term "set" (e.g., "a set of items") or "subset" unless otherwise noted or contradicted by context, is to be construed as a nonempty collection comprising one or more members. Further, unless otherwise noted or contradicted by context, the term "subset" of a corresponding set does not necessarily denote a proper subset of the corresponding set, but subset and corresponding set may be equal.

Conjunctive language, such as phrases of form "at least one of A, B, and C," or "at least one of A, B and C," unless specifically stated otherwise or otherwise clearly contradicted by context, is otherwise understood with context as used in general to present that an item, term, etc., may be either A or B or C, or any nonempty subset of set of A and B and C. For instance, in illustrative example of a set having three members, conjunctive phrases "at least one of A, B, and C" and "at least one of A, B and C" refer to any of following sets: {A}, {B}, {C}, {A, B}, {A, C}, {B, C}, {A, B, C}. Thus, such conjunctive language is not generally intended to imply that certain examples require at least one of A, at least one of B and at least one of C each to be present. In addition, unless otherwise noted or contradicted by context, the term "plurality" indicates a state of being plural (e.g., "a plurality of items" indicates multiple items). In at least one example, the number of items in a plurality is at least two, but can be more when so indicated either explicitly or by context. Further, unless stated otherwise or otherwise clear from context, the phrase "based on" means "based at least in part on" and not "based solely on."

Operations of processes described herein can be performed in any suitable order unless otherwise indicated herein or otherwise clearly contradicted by context. In at least one example, a process such as those processes described herein (or variations and/or combinations thereof) is performed under control of one or more computer systems configured with executable instructions and is implemented as code (e.g., executable instructions, one or more computer programs or one or more applications) executing collectively on one or more processors, by hardware or combinations thereof. In at least one example, code is stored on a computer-readable storage medium, for example, in the form of a computer program comprising a plurality of instructions executable by one or more processors. In at least one example, a computer-readable storage medium is a non-transitory computer-readable storage medium that excludes transitory signals (e.g., a propagating transient electric or electromagnetic transmission) but includes non-transitory data storage circuitry (e.g., buffers, cache, and queues) within transceivers of transitory signals. In at least one example, code (e.g., executable code or source code) is stored on a set of one or more non-transitory computer-readable storage media having stored thereon executable instructions (or other memory to store executable instructions) that, when executed (i.e., as a result of being executed) by one or more processors of a computer system, cause computer system to perform operations described herein. In at least one example, set of non-transitory computer-readable storage media comprises multiple non-transitory computer-readable storage media and one or more of individual non-transitory storage media of multiple non-transitory computer-readable storage media lack all of code while multiple non-transitory computer-readable storage media collectively store all of code. In at least one example, executable instructions are executed such that different instructions are executed by different processors - for example, a non-transitory computer-readable storage medium store instructions and a main central processing unit ("CPU") executes some of instructions while a graphics processing unit ("GPU") executes other instructions. In at least one example, different components of a computer system have separate processors and different processors execute different subsets of instructions.

Accordingly, in at least one example, computer systems are configured to implement one or more services that singly or collectively perform operations of processes described herein and such computer systems are configured with applicable hardware and/or software that enable performance of operations. Further, a computer system that implements at least one example of present disclosure is a single device and, in another example, is a distributed computer system comprising multiple devices that operate differently such that a distributed computer system performs operations described herein and such that a single device does not perform all operations.

Use of any and all examples, or exemplary language (e.g., "such as") provided herein, is intended merely to better illuminate examples of disclosure and does not pose a limitation on scope of disclosure unless otherwise claimed. No language in specification should be construed as indicating any non-claimed element as essential to practice of disclosure.

All references, including publications, patent applications, and patents, cited herein are hereby incorporated by reference to the same extent as if each reference were individually and specifically indicated to be incorporated by reference and were set forth in its entirety herein.

In description and claims, terms "coupled" and "connected," along with their derivatives, may be used. It should be understood that these terms may be not intended as synonyms for each other. Rather, in particular examples, "connected" or "coupled" may be used to indicate that two or more elements are in direct or indirect physical or electrical contact with each other. "Coupled" may also mean that two or more elements are not in direct contact with each other, but yet still co-operate or interact with each other.

Unless specifically stated otherwise, it may be appreciated that throughout specification terms such as "processing," "computing," "calculating," "determining," or like, refer to action and/or processes of a computer or computing system, or similar electronic computing device, that manipulate and/or transform data represented as physical, such as electronic, quantities within computing system's registers and/or memories into other data similarly represented as physical quantities within computing system's memories, registers or other such information storage, transmission or display devices.

In a similar manner, the term "processor" may refer to any device or portion of a device that processes electronic data from registers and/or memory and transforms that electronic data into other electronic data that may be stored in registers and/or memory. As non-limiting examples, "processor" may be a CPU or a GPU. A "computing platform" may comprise one or more processors. As used herein, "software" processes may include, for example, software and/or hardware entities that perform work overtime, such as tasks, threads, and intelligent agents. Also, each process may refer to multiple processes, for carrying out instructions in sequence or in parallel, continuously or intermittently. In at least one example, terms "system" and "method" are used herein interchangeably insofar as a system may embody one or more methods and methods may be considered a system.

In the present document, references may be made to obtaining, acquiring, receiving, or inputting analog or digital data into a subsystem, computer system, or computer-implemented machine. In at least one example, the process of obtaining, acquiring, receiving, or inputting analog and digital data can be accomplished in a variety of ways such as by receiving data as a parameter of a function call or a call to an application programming interface. In at least one example, processes of obtaining, acquiring, receiving, or inputting analog or digital data can be accomplished by transferring data via a serial or parallel interface. In at least one example, processes of obtaining, acquiring, receiving, or inputting analog or digital data can be accomplished by transferring data via a computer network from providing entity to acquiring entity. In at least one example, references may also be made to providing, outputting, transmitting, sending, or presenting analog or digital data. In various examples, processes of providing, outputting, transmitting, sending, or presenting analog or digital data can be accomplished by transferring data as an input or output parameter of a function call, a parameter of an application programming interface or interprocess communication mechanism.

Although descriptions herein set forth example implementations of described techniques, other architectures may be used to implement described functionality, and are intended to be within scope of this disclosure. Furthermore, although specific distributions of responsibilities may be defined above for purposes of description, various functions and responsibilities might be distributed and divided in different ways, depending on circumstances.

Furthermore, although subject matter has been described in language specific to structural features and/or methodological acts, it is to be understood that subject matter claimed in appended claims is not necessarily limited to specific features or acts described. Rather, specific features and acts are disclosed as exemplary forms of implementing the claims.

While various embodiments of the present disclosure have been described in detail, it is apparent that modifications and alterations of those embodiments will occur to those skilled in the art. However, it is to be expressly understood that such modifications and alterations are within the scope and spirit of the present disclosure. Further, the invention(s) described herein are capable of other embodiments and of being practiced or of being carried out in various ways. In addition, it is to be understood that the phraseology and terminology used herein is for the purposes of description and should not be regarded as limiting.

The foregoing examples of the present disclosure have been presented for purposes of illustration and description. These examples are not intended to limit the disclosure to the form disclosed herein. Consequently, variations and modifications commensurate with the teachings of the description of the disclosure, and the skill or knowledge of the relevant art, are within the scope of the present disclosure. The specific embodiments described in the examples provided herein are intended to further explain the best mode known for practicing the disclosure and to enable others skilled in the art to utilize the disclosure in such, or other, embodiments and with various modifications required by the particular applications or uses of the present disclosure. It is intended that the appended claims be construed to include alternative embodiments to the extent permitted by the prior art.

**Claims**

1. A computer-implemented method for providing fingernail measurements, the computer-implemented method comprising:

   receiving first image data of a primary view of at least one fingernail;
   receiving second image data of a secondary view of the at least one fingernail;
   generating a three-dimensional (3D) digital nail model using the first and second image data; and
   determining a plurality of nail measurements based on the 3D digital nail model.

2. The computer-implemented method according to claim 1, wherein the first and second image data is received using a camera, and wherein the first and second image data is received in the form of a video or a plurality of images at different angles and at different distances relative to the camera.

3. The computer-implemented method according to claim 1 or 2, wherein the 3D digital nail model comprises a plurality of pixels to represent the at least one fingernail, and wherein the plurality of nail measurements are determined based on the plurality of pixels.

4. The computer-implemented method according to one of the preceding claims, wherein the 3D digital nail model is generated using point cloud modeling.

5. The computer-implemented method according to one of the preceding claims, wherein the plurality of nail measurements are determined using 2D image segmentation.

6. The computer-implemented method according to one of the preceding claims, wherein the primary view comprises a top-down view of at least one fingernail.

7. The computer-implemented method according to one of the preceding claims, wherein the secondary view comprises a between top-down and straight-on angled view of the nail plate of at least one fingernail.

8.   The computer-implemented method according to one of the preceding claims, wherein the first and second image data are taken with a reference object.

9.   The computer-implemented method according to one of the preceding claims, wherein the reference object comprises an object of standard size, including, but not limited to a credit card, a coin, etc.

10.  The computer-implemented method according to one of the preceding claims, wherein the plurality of nail measurements comprises a curvature of a nail plate of at least one fingernail.

11.  The computer-implemented method according to one of the preceding claims, wherein the plurality of nail measurements comprises a shape of a nail plate of at least one fingernail.

12.  The computer-implemented method according to one of the preceding claims, further comprising:
     displaying the plurality of nail measurements to a user interface of a mobile device running the native mobile application.

13.  The computer-implemented method according to one of the preceding claims, further comprising:
     transmitting the plurality of nail measurements to another device.

14.  A computer-implemented method for providing fingernail measurements via a native mobile application, the computer-implemented method comprising:

     receiving first image data of a primary view of at least one fingernail;
     receiving second image data of a secondary view of the at least one fingernail;
     generating a three-dimensional (3D) digital nail model using the first and second image data; and
     determining a plurality of nail measurements based on the 3D digital nail model.

15.  A, preferably mobile, device configured to carry out a method according to one of the preceding claims and/or comprising:

     a memory;
     a camera; and
     a processor, when executing a mobile application, the mobile device configured to:

          obtain first image data of a primary view of a user's fingernail nail plate;
          obtain second image data of a secondary view of the user's fingernail nail tip;
          generate a three-dimensional (3D) digital nail model using the first and second image data; and
          determine a plurality of nail measurements based on the 3D digital nail model.

16.  A user interface (UI) for nail measurement according to a method of one of claims 1 to 14.

17.  A mobile application configured to, when being executed by a computer, in particular a mobile device, carry out a method according to one of claims 1 to 14.

FIG. 1

FIG. 2

FIG. 3

| Correct detection | Incorrect detection without perspective correction | Incorrect detection with perspective correction |

FIG. 4

FIG. 5

FIG. 6

FIG. 7

FIG. 8

FIG. 9

FIG. 10

START

305 —

Receive first image data of a primary view of at least one fingernal

310 —

Receive second image data of a secondary view of the at least one fingernail

315 —

Generate a 3D digital nail model using the first and second image data

320 —

Determining a plurality of nail measurements based on the generated 3D digital nail model

End

300

FIG. 11

FIG. 12

4:25  ◁ ⬜ Left Hand ↻

Now show your left hand's fingers...

141 x 199

4:25

×

Set up a reference object
A standard-size card is
needed for reference.
You can use a
Gift card
Library card
Loyalty card
Credit card

Get started

FIG. 13

Europäisches
Patentamt

European
Patent Office

Office européen
des brevets

# EUROPEAN SEARCH REPORT

Application Number

EP 23 19 4315

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KR 2023 0026217 A (BEAUTYID CO LTD [KR]) 24 February 2023 (2023-02-24) | 1-12,15 | INV. G06T7/55 |
| Y | * the whole document * | 13-17 | G06T7/60 |
| Y | US 2023/124480 A1 (FARRAN GEORGINA [GB] ET AL) 20 April 2023 (2023-04-20) * the whole document * | 13-17 | ADD. A45D31/00 G01B11/00 G06F3/048 |

TECHNICAL FIELDS SEARCHED (IPC)

G06T
G06F
G01B
A45F
G06V
A45D

The present search report has been drawn up for all claims

| Place of search | Date of completion of the search | Examiner |
|---|---|---|
| Munich | 11 February 2024 | Wu, Zifeng |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 23 19 4315

This annex lists the patent family members relating to the patent documents cited in the above-mentioned European search report.
The members are as contained in the European Patent Office EDP file on
The European Patent Office is in no way liable for these particulars which are merely given for the purpose of information.

11-02-2024

| Patent document cited in search report | Publication date | Patent family member(s) | Publication date |
|---|---|---|---|
| KR 20230026217 A | 24-02-2023 | NONE | |
| US 2023124480 A1 | 20-04-2023 | US 2023124480 A1 | 20-04-2023 |
| | | WO 2023062374 A1 | 20-04-2023 |

EPO FORM P0459

For more details about this annex : see Official Journal of the European Patent Office, No. 12/82